# EUROPÄISCHE PATENTANMELDUNG

(11) **EP 2 067 449 A1**
(43) Veröffentlichungstag der Anmeldung: **10.06.2009**
(21) Anmeldenummer: 07122134.5
(22) Anmeldetag: 03.12.2007
(51) Int. Cl.: A61B 19/00, A61B 17/88, A61B 17/86

(54) **Befestigungssystem und Befestigungsverfahren für Referenzanordnungen**

(71) Anmelder: BrainLAB AG, 85622 Feldkirchen (DE)
(72) Erfinder: Birkenbach, Rainer, 85435 Erding (DE); Immerz, Martin, 82166 Gräfelfing (DE); Millahn, Manuel, 80799 München (DE)
(74) Vertreter: Schwabe - Sandmair - Marx

(57) **Zusammenfassung**

Befestigungssystem für Referenzanordnungen, wobei ein verfestigbares Material (1) eine der Referenzanordnung zugeordnete Ansatzbasis (3) gegenüber einem Knochen (4) fixiert.

## Beschreibung

Die Erfindung betrifft ein Befestigungssystem bzw. ein Befestigungsverfahren für Referenzanordnungen, die an einem Knochen angebracht werden, um dessen Ortung und Verfolgung mit Hilfe eines medizintechnischen Navigations- bzw. Trackingsystems zu ermöglichen.

Bisher wurden solche Referenzanordnungen an dem zu verfolgenden Knochen durch Verschraubungen angebracht. Die dazu verwendeten Schrauben weisen ein selbstschneidendes Gewinde auf, welches sich beim Eindrehen der Schraube in die Knochensubstanz einschneidet. Hierbei wird kein Loch in die Knochensubstanz vorgebohrt, so dass letztendlich der Knochen beim Anbringen der Referenzanordnungen hohen mechanischen Belastungen ausgesetzt ist. Problematisch ist dies insbesondere bei älteren Patienten, deren Knochen keine dementsprechend hohe Belastungsgrenze aufweisen. Auch kann bei einer bikortikalen Anbringung der Referenzanordnung, bei welcher der Knochen-Kortex an zwei zumeist nahe liegenden Stellen am Knochen durch die Anbringung einer Haltevorrichtung für die Referenzanordnung durchdrungen wird, die Knochenstruktur derart geschwächt werden, dass es zu post-operativen oder intra-operativen Frakturen während der Implantateinbringung kommen kann. So kann es vorkommen, dass beim Befestigen der Referenzanordnungen durch Schrauben Knochenstücke am Ort der Verschraubung ausbrechen oder schlimmstenfalls der Knochen an dieser Stelle bricht.

Es ist die Aufgabe der Erfindung ein Befestigungssystem bzw. ein Befestigungsverfahren für Referenzanordnungen bereitzustellen, mit dem Referenzanordnungen schonend am Knochen angebracht werden können.

Diese Aufgabe wird durch ein Befestigungssystem gemäß dem Anspruch 1 und ein Befestigungsverfahren gemäß dem Anspruch 15 gelöst. Die Unteransprüche definieren bevorzugte Ausführungsformen der Erfindung.

Bei dem erfindungsgemäßen Befestigungssystem wird eine Ansatzbasis, die einer Referenzanordnung zugeordnet, sprich an ihr befestigt werden kann, mittels eines verfestigbaren Materials gegenüber dem Knochen fixiert. Dabei kann primär zwischen Materialien unterschieden werden, die lediglich verfestigbar sind, und Materialien, die zusätzlich wieder verflüssigbar sind. Bei zweiteren wäre es daher grundsätzlich möglich, das Material mehrmals zu verfestigen und anschließend wieder zu verflüssigen. Bei dem Material handelt es sich also um eine Art Klebstoff, welche den Kraftschluss zwischen Knochen und Ansatzbasis herstellt. Mit anderen Worten wird die Ansatzbasis mit dem Knochen verklebt. Zu diesem Zwecke sind folgende Materialien denkbar: Polymere, Polymerkeramik, Zement, Metall, Glas, Knochenzement, Keramik (AL₂O₃, TiO₂, ZrO₂), Metallpulver (Ti, Fe) und Glas (SiO₂).

In einer bevorzugten Ausführungsform liegt das verfestigbare Material anfangs in einem viskosen Zustand vor und wird, sobald es in den Bereich verbracht worden ist, an dem die "Verklebung" zwischen Knochen und Ansatzbasis stattfinden soll, verfestigt bzw. ausgehärtet. Dieser Bereich befindet sich im Allgemeinen zwischen der Ansatzbasis und dem Knochen. Dabei kann das Aushärten selbstständig, d.h. ohne äußere Einflüsse stattfinden oder erst durch Aufbringen äußerer Einflussgrößen auf das Material hervorgerufen werden. Denkbar dabei wäre beispielsweise von außen aufgebrachte Wärme, UV-Strahlung oder ähnliches. Das Material ist nach dem Verfestigen tragfähig bzw. belastbar, so dass die Ansatzbasis und somit auch die Referenzanordnung gegenüber dem Knochen fixiert wird und sich relativ zu diesem nicht mehr bewegen kann.

Grundsätzlich ist es auch denkbar, dass anstelle einer Ansatzbasis für Referenzanordnungen auch ein dem Knochen zugehöriges Knochenstück an diesen fixiert wird, so dass beispielsweise nach einem Knochenbruch die Knochenstücke miteinander "verklebt" werden.

Ferner ist es denkbar, dass das Material in einem Teil der Ansatzbasis, beispielsweise einem Reservoir der Ansatzbasis vorliegt, bevor es in den Bereich zwischen Ansatzbasis und Knochen verbracht wird, an dem es letztendlich aushärtet. So muss zusätzlich zur Ansatzbasis kein Material extern bereitgestellt werden, was die Handhabung des Befestigungssystems wesentlich vereinfacht. Das Material kann beispielsweise, nachdem sich die Ansatzbasis an einer gewünschten Stelle am Knochen befindet, aus einem Reservoir der Ansatzbasis ausgebracht werden, um sich letztendlich im Bereich zwischen Ansatzbasis und Knochen zu verfestigen. Dabei kann das Material etwa durch einen Kolben aus der Ansatzbasis herausgedrückt werden oder, wenn es in einem festen Zustand in der Ansatzbasis vorliegt, verflüssigt werden und aus der Ansatzbasis herauslaufen oder herausgedrückt werden, um letztendlich im Bereich zwischen Ansatzbasis und Knochen verfestigt zu werden.

Es ist jedoch auch denkbar, dass das Material "von außen" in den Bereich zwischen Ansatzbasis und Knochen eingebracht wird, etwa durch eine Art Klebeinstrument, welches das Material bei Betätigung freigibt.

Gemäß einer bevorzugten Ausführungsform ist das verfestigbare Material zusätzlich durch einen äußeren Einfluss verflüssigbar, sobald diese auf das Material aufgebracht wird. So kann sich das Material beispielsweise durch Ultraschallwellen, thermische Energie, Infrarotstrahlung, Mikrowellenstrahlung oder UV-Strahlung verflüssigen, sobald diese beispielsweise durch ein gesondertes Gerät auf das Material aufgebracht wird.

Ferner sind grundsätzlich zwei Arten einer Ansatzbasis denkbar.

Bei der ersten Art wird die Ansatzbasis lediglich auf den Knochen aufgesetzt, wobei das Material zwischen der Knochenoberfläche und der Ansatzbasis angeordnet ist. Um die Haftung des Materials auf der Knochenoberfläche zu verbessern, kann die Knochenoberfläche vor dem Aufsetzen der Ansatzbasis durch geeignete Mittel aufgeraut werden. Somit ist keine weitere Bearbeitung an der Knochensubstanz nötig. Dies kann insbesondere an Knochen von Vorteil sein, welche schon bei geringen mechanischen Belastungen zu brechen drohen.

Bei der zweiten Art der Ansatzbasis ragt diese durch den Knochen-Kortex in die Knochen-Spongiosa ein. Dies wird insbesondere dadurch erreicht, dass in den Knochen eine Vertiefung erzeugt wird, beispielsweise ein Sackloch in den Knochen gebohrt wird, in welche die Ansatzbasis eingesetzt wird. Das Material kann dabei vor oder nach dem Einsetzen der Ansatzbasis in den Bereich zwischen Ansatzbasis und Knochen eingebracht werden, so dass die Ansatzbasis im Knochen gänzlich von verfestigbarem Material umgeben ist. Dadurch wird zusätzliche Festigkeit der "Verklebung" erreicht, da das verfestigbare Material auch in die poröse Knochen-Spongiosa eindringen und sich mit diesem formschlüssig verbinden kann.

Da die Ansatzbasis in der Spongiosa endet und den Knochen-Kortex lediglich an einer Stelle durchdringt, spricht man hierbei von einer unikortikalen Anbringung der Absatzbasis. Entgegen der vorliegenden Erfindung werden im Stand der Technik Referenzanordnungen oftmals "bikortikal" angebracht, wobei der Knochen-Kortex auf zwei sich gegenüberliegenden Seiten des Knochens durch Teile der Referenzanordnung durchdrungen wird. Dies führt zwar zu einer erhöhten Belastung des Knochengewebes, war jedoch nötig, da sich eine unikortikale Verschraubung bei Gebrauch zu lösen droht. Aufgrund der formschlüssigen Verhebung der Ansatzbasis mit Bereichen der Songiosa ist dies höher belastbar und daher eine Lockerung der Ansatzbasis vom Knochen nicht zu befürchten.

Bei einer weiteren Ausführungsform ist es jedoch denkbar, dass das Material lediglich in einem Grundbereich des Sackloches im Knochen vorliegt, so dass sich die Ansatzbasis nach Gebrauch wieder leicht vom Knochen lösen lässt.

Ferner ist es denkbar, dass die Ansatzbasis einen Kanal aufweist, durch den das Material durch zumindest eine Austrittsöffnung in den Bereich zwischen Ansatzbasis und Knochen verbracht werden kann. Dies ist insbesondere in Verbindung mit einem Reservoir für das Material in der Ansatzbasis vorstellbar, da eine solche Ausgestaltungsform die Handhabung der Ansatzbasis wesentlich erleichtert. Dabei ist es ferner denkbar, dass der Kanal innerhalb der Ansatzbasis an seiner Innenwandung Vorstände oder Ausnehmungen aufweist. Wenn sich das Material sowohl im Bereich zwischen Ansatzbasis und Knochen, als auch durch die Austrittsöffnung des Kanals hindurch bis in den Kanal hinein verfestigt, kann ein Formschluss der Ansatzbasis an den Knochen durch die Vorstände oder Ausnehmungen an der Kanalinnenwandung gewährleistet werden. Falls nun die Ansatzbasis außer innerhalb des Kanals keine Bindung durch das Material erfährt (beispielsweise durch eine glatte Außenwandung der Ansatzbasis), kann ein Lösen nach Gebrauch der Ansatzbasis vom Knochen dadurch geschehen, dass das Material innerhalb des Kanals entfernt wird. So kann bei einem zylindrischen Kanal mit Vorständen oder Ausnehmungen durch Ausbohren des Materials aus der Ansatzbasis das Lösen der Ansatzbasis vom Knochen bewirkt werden.

Es können jedoch auch an der Außenwandung der Ansatzbasis Vorstände oder Ausnehmungen ausgebildet sein, die einen Formschluss der Ansatzbasis über das Material mit dem Knochen bewirken.

Ferner kann die Ansatzbasis so ausgestaltet sein, dass die Referenzanordnung von der Ansatzbasis abnehmbar ist. So kann die Ansatzbasis unabhängig von der Referenzanordnung am Knochen angebracht und auf diesem wieder gelöst werden. Lediglich für den Gebrauch in Verbindung mit einem Navigations- bzw. Trackingsystems ist die Referenzanordnung an der Ansatzbasis angebracht.

Die Erfindung betrifft ferner ein Verfahren zum Befestigen einer Referenzanordnung an einem Knochen mit einem verflüssigbaren Material. Hierbei wird die Ansatzbasis in oder an einem Knochen wie schon oben beschrieben angesetzt. Das Material kann dabei schon in einem flüssigen Zustand vorliegen oder wird erst durch Ultraschall, thermische Energie, Infrarotstrahlung, Mikrowellenstrahlung oder UV-Strahlung verflüssigt, um in einen Bereich zwischen Ansatzbasis und Knochen verbracht zu werden. Die Zuführung des Materials für den Bereich kann auch über einen in der Ansatzbasis ausgebildeten Kanal geschehen. Anschließend härtet das Material entweder durch äußere Einflüsse oder selbstständig aus und fixiert so die Ansatzbasis am Knochen. Nachdem die an der Ansatzbasis angebrachte Referenzanordnung ihren Zweck in Verbindung mit einem Navigations- bzw. Trackingsystems erfüllt hat, kann die Ansatzbasis wieder vom Knochen entfernt werden. Dies kann einerseits durch Wiederverflüssigen des Materials geschehen oder durch mechanische Entfernung des Materials, beispielsweise durch Ausbohren des Materials aus dem Zuführungskanal in der Ansatzbasis. Das Wiederverflüssigen des Materials kann dabei durch die gleichen Einflüsse geschehen, wie schon oben beschrieben. Auch ist es denkbar, dass die Ansatzbasis teilweise im oder am Knochen verbleibt, wobei der überstehende bzw. störende Teil der Ansatzbasis mechanisch abgetrennt wird, so dass der verbleibende Rest der Ansatzbasis bündig mit dem Knochen abschließt. Dies kann insbesondere in Verbindung mit einer Ansatzbasis geschehen, die aus einem biologisch abbaubaren Material besteht und im Laufe der Zeit abgebaut wird bzw. mit dem Knochen verwächst. Für die Ansatzbasis ist jedoch auch Metall oder Knochengewebe als Werkstoff denkbar. Die Erfindung wird anhand der beiliegenden Figuren in einzelnen Ausführungsformen näher erläutert, wobei sie die hierin beschriebenen Merkmale einzeln sowie in jedweder sinnvollen Kombination umfassen kann. Es ist denkbar, dass eine Ansatzbasis aus geeignetem Material (beispielsweise Metall) sowohl als Werkzeug bzw. Bohrer zum Erzeugen eines Sacklochs im Knochen ausgebildet ist gleichzeitig auch hierin beschriebene Merkmale aufweist. Somit kann die Ansatzbasis gleichzeitig zum Bohren des Sacklochs und zum Fixieren der Referenzanordnung genutzt werden. Es zeigen:
- Figur 1: eine Ansatzbasis mit strukturierter Außenfläche,
- Figur 2: eine Ansatzbasis mit einem Zuführkanal und Austrittsöffnungen für das Material,
- Figur 3: eine Ansatzbasis mit strukturierter Innenwandung des Zuführkanals,
- Figur 4: eine perspektivische Ansicht einer Ansatzbasis,
- Figur 5: eine bündig mit der Oberfläche abschließende Ansatzbasis mit Zuführkanal und Austrittsöffnung,
- Figur 6: eine Ansatzbasis zum Aufsetzen auf die Knochenoberfläche mit mehreren Zuführkanälen,
- Figur 7: eine Ansatzbasis zum Aufsetzen auf die Knochenoberfläche mit einem Zuführkanal,
- Figur 8: eine in Material eingebettete Ansatzbasis an der Knochenoberfläche,
- Figur 9: eine Ansatzbasis mit daran befestigter Referenzanordnung, und
- Figur 10: eine Ansatzbasis mit von ihr lösbarer Referenzanordnung.

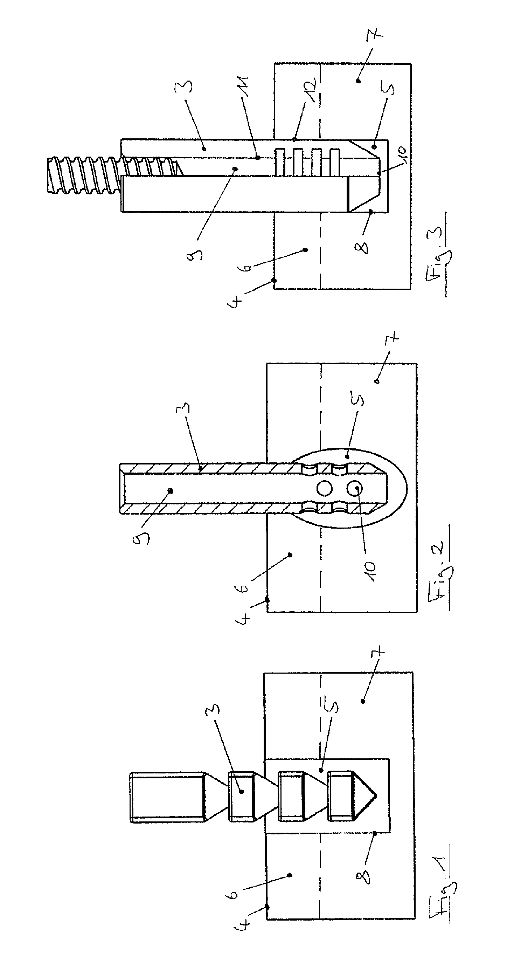

In der Figur 1 ist eine Ansatzbasis 3 zu sehen, welche in ein in einen Knochen 4 gebohrtes Sackloch 8 eingesetzt wird. Die Ansatzbasis 3 erstreckt sich mit dem Sackloch 8 durch den Knochen-Kortex 6 in die Knochen-Spongiosa 7 hinein. Dabei weist das Sackloch 8 einen geringfügig geringeren Durchmesser auf als die Ansatzbasis 3, so dass ein Bereich 5 entsteht, in den das Material eingebracht werden kann. Dabei weist die Ansatzbasis 3 eine strukturierte Oberfläche in Form von radial umlaufenden Nuten auf, die den Formschluss zwischen Knochen 4 und Ansatzbasis 3 sicherstellen.

In der Figur 2 ist eine Ansatzbasis 3 zu sehen, welche einen Kanal 9 aufweist, durch den das Material in den Bereich 5 verbracht werden kann. Dazu weist die Ansatzbasis 3 an ihrem im Knochen 4 sitzenden Ende Öffnungen 10 auf, durch die das Material austreten kann. Der Bereich 5 erstreckt sich somit in die Knochen-Spongiosa 7 hinein, wobei ein Formschluss durch vorliegendes Material in der Knochen-Spongiosa 7 und in den Austrittsöffnungen 10 bewerkstelligt wird.

Daher ist auch keine separate strukturierte Außenfläche der Ansatzbasis 3 nötig.

In der Figur 3 ist eine Ansatzbasis 3 gezeigt, deren Außendurchmesser ebenso groß ist wie der des Sacklochs 8. Somit liegt die Ansatzbasis 3 direkt am Knochen 4 an, so dass das Material lediglich im Bereich 5 im unteren Bereich des Sacklochs 8 vorliegen kann, sich jedoch durch die Austrittsöffnung 10 in den Kanal 9 der Ansatzbasis hineinerstreckt. Dabei weist der Kanal 9 mehrere radial umlaufende Nuten auf, die den Formschluss der Ansatzbasis 3 mit dem Knochen 4 sicherstellen.

Am oberen Ende der Ansatzbasis 3 ist zusätzlich ein Bohrinstrument gezeigt, welches zum Lösen der Ansatzbasis 3 von oben in den Kanal 9 eingeführt werden kann und das Material mechanisch entfernt. Nachdem die umlaufenden Nuten an der Innenwandung des Kanals 9 vom Material befreit sind, kann die Ansatzbasis 3 aus dem Knochen 4 entfernt werden.

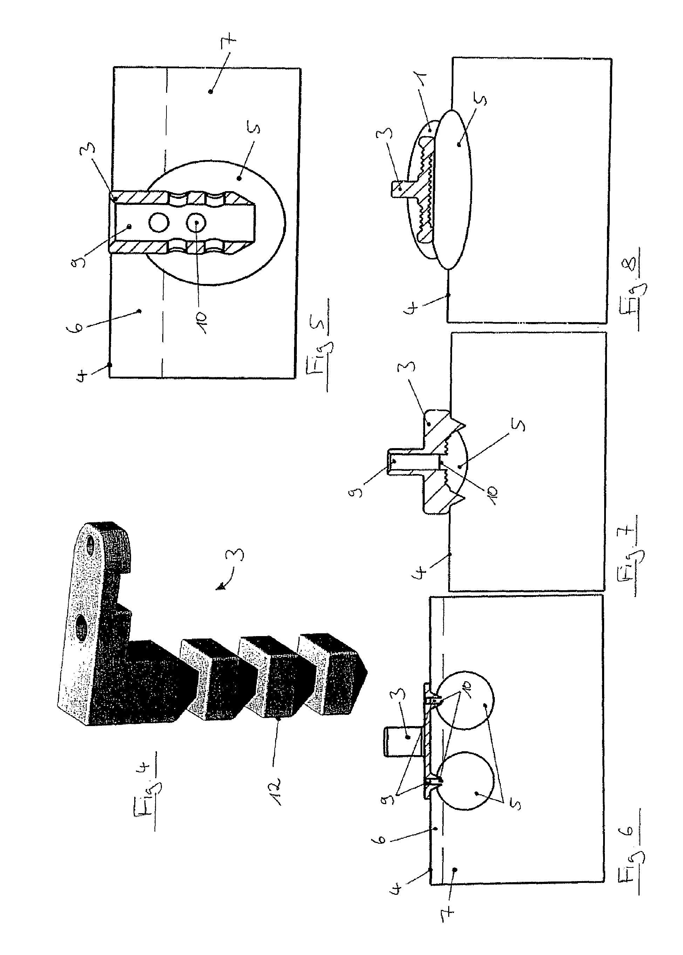

In der Figur 4 ist eine Ansatzbasis 3, ähnlich wie die Ansatzbasis 3 aus Figur 1 gezeigt, welche an ihrem oberen Ende zusätzlich eine Aufnahme für eine Referenzanordnung aufweist. So kann die Referenzanordnung 2 von der Ansatzbasis 3 gelöst werden.

In der Figur 5 ist eine mit der Oberfläche des Knochens 4 bündig abschließende Ansatzbasis 3 gezeigt, die die gleichen Merkmale aufweist wie die Ansatzbasis 3 aus Figur 2. Jedoch ist diese so verkürzt, dass sie nach Gebrauch im Knochen 4 verbleiben kann, da sie keinen störenden Vorstand am Knochen 4 bildet.

Figur 6 zeigt eine Ansatzbasis 3, welche lediglich auf die Oberfläche des Knochens 4 aufgesetzt wird. In einem tellerförmigen Abschnitt sind mehrere Kanäle 9 ausgebildet, durch welche das Material 1 in die Bereiche 5 verbracht werden kann, um somit einen Formschluss der Ansatzbasis 3 mit dem Knochen 4 zu erzeugen. Dabei erstrecken sich die Fortsätze mit den Kanälen 9 und den Öffnungen 10 durch den Knochen-Kortex 6 in die Spongiosa 7, so dass das Material nach Verbringung in der Spongiosa 7 angeordnet ist.

Figur 7 zeigt eine Ansatzbasis 3 zum Aufsetzen auf eine Oberfläche eines Knochens 4, wobei die Unterseite der Ansatzbasis 3 strukturiert ist, um einen sicheren Formschluss der Ansatzbasis 3 mit dem Knochen 4 zu gewährleisten. Dabei wird das Material durch den Kanal 9 der Ansatzbasis 3 aus der Öffnung 10 in den Bereich 5 zwischen Ansatzbasis 3 und Knochen 4 verbracht. So liegt das Material lediglich zwischen Ansatzbasis 3 und Knochen 4 vor, dringt jedoch nicht in die Spongiosa 7 ein.

In der Figur 8 ist eine Ansatzbasis 3 zu sehen, welche auf einen zuvor mit Material beaufschlagten Bereich 5 an der Oberfläche des Knochens 4 aufgesetzt wird und durch anschließendes Aufbringen von weiteren Materials 1 auf den Bereich 5 und die Ansatzbasis 3 am Knochen 4 fixiert wird.

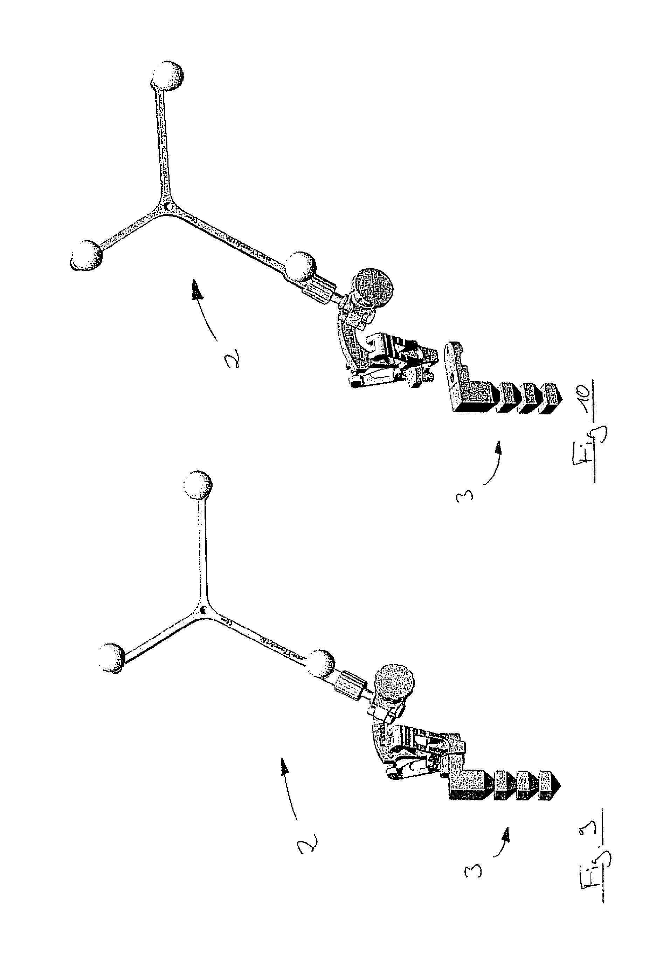

Figur 9 zeigt eine perspektivische Ansicht einer Ansatzbasis 3 aus Figur 1 bzw. 4 wobei eine Referenzanordnung 2 mit drei Reflektoren am oberen Ende der Ansatzbasis 3 befestigt ist. Die Position und Ausrichtung der Referenzanordnung 2 bezüglich der Ansatzbasis 3 kann dabei durch ein zwischen Ansatzbasis 3 und Referenzanordnung 2 angeordnetes verstellbares Bindeglied variiert werden.

Figur 10 zeigt die Referenzanordnung 2 mit der Ansatzbasis 3 aus Figur 9, wobei die Referenzanordnung 2 von der Ansatzbasis 3 gelöst ist.

## Patentansprüche

1. Befestigungssystem für Referenzanordnungen, **dadurch gekennzeichnet, dass** ein verfestigbares Material (1) eine der Referenzanordnung (2) zugeordnete Ansatzbasis (3) gegenüber einem Knochen (4) fixiert.

2. Befestigungssystem für Referenzanordnungen gemäß Anspruch 1, **dadurch gekennzeichnet, dass** das verfestigbare Material (1), während es in einen an Ansatzbasis (3) und Knochen (4) anliegenden Bereich (5) verbracht wird, in viskoser Form vorliegt und, nachdem es in dem Bereich (5) zum Liegen kommt, einen festen, insbesondere tragfähigen Zustand annimmt, um die Ansatzbasis (3) gegenüber dem Knochen (4) zu fixieren.

3. Befestigungssystem für Referenzanordnungen gemäß einem der Ansprüche 1 oder 2, **dadurch gekennzeichnet, dass** das Material (1) vor dem Verbringen in den Bereich (5) in einem Teil der Ansatzbasis (3) vorliegt.

4. Befestigungssystem für Referenzanordnungen gemäß einem der Ansprüche 1 oder 2, **dadurch gekennzeichnet, dass** das Material (1) vor dem Verbringen in den Bereich (5) außerhalb der Ansatzbasis (3) vorliegt.

5. Befestigungssystem für Referenzanordnungen gemäß einem der vorhergehenden Ansprüche, **dadurch gekennzeichnet, dass** das Material
(1) durch eine äußere, auf das Material (1) aufgebrachte Größe verflüssigbar ist, insbesondere durch eine oder mehrere der folgenden:
- Ultraschallwellen
- Thermische Energie
- Infrarotstrahlung
- Mikrowellenstrahlung
- UV-Strahlung

6. Befestigungssystem für Referenzanordnungen gemäß einem der vorhergehenden Ansprüche, **dadurch gekennzeichnet, dass** die Ansatzbasis (3) auf den Knochen (4) aufgesetzt wird und der an Ansatzbasis (3) und Knochen (4) anliegende Bereich (5) im wesentlichen an einem Teil der Außenseite eines Knochen-Cortex (6) angeordnet ist.

7. Befestigungssystem für Referenzanordnungen gemäß einem der Ansprüche 1 bis 5, **dadurch gekennzeichnet, dass** sich die Ansatzbasis (3) durch den Knochen-Cortex (6) in die Knochen-Spongiosa (7), insbesondere in ein im Knochen (4) vorgebohrtes Sackloch (8) hinein erstreckt und der an Ansatzbasis (3) und Knochen (4) anliegende Bereich (5) um die Ansatzbasis (3) angeordnet ist.

8. Befestigungssystem für Referenzanordnungen gemäß dem vorhergehenden Anspruch, **dadurch gekennzeichnet, dass** sich der an Ansatzbasis (3) und Knochen (4) anliegende Bereich (5) ein Grundbereich des im Knochen vorgebohrten Sackloches (8) ist.

9. Befestigungssystem für Referenzanordnungen gemäß einem der vorhergehenden Ansprüche, **dadurch gekennzeichnet, dass** sich durch die Ansatzbasis (3), insbesondere entlang einer Längsachse der Ansatzbasis (3) ein Kanal (9) erstreckt, wobei sich an den Kanal (9) zumindest eine Austrittsöffnung (10) anschließt, um das viskose Material (1) zu dem Bereich (5) zu leiten, in dem es sich anschließend verfestigt.

10. Befestigungssystem für Referenzanordnungen gemäß dem vorhergehenden Anspruch, **dadurch gekennzeichnet, dass** die Innenwandung (11) des Kanals (9) zumindest eine Ausnehmung oder Einschnürung, insbesondere mindestens eine in der Innenwandung (11) radial umlaufende Nut aufweist und die Außenwandung (12) der Ansatzbasis (3) eine im Wesentlichen glatte Oberfläche aufweist.

11. Befestigungssystem für Referenzanordnungen gemäß einem der Ansprüche 1 bis 9, **dadurch gekennzeichnet, dass** die Außenwandung (12) der Ansatzbasis (3) zumindest eine Ausnehmung, Einschnürung oder Erhebung, insbesondere mindestens eine an der Außenwandung (12) radial umlaufende Nut oder Kragen aufweist.

12. Befestigungssystem für Referenzanordnungen gemäß einem der vorhergehenden Ansprüche, **dadurch gekennzeichnet, dass** sich das viskose Material (1) vor der Verfestigung zumindest teilweise in die Knochen-Spongiosa (7) hinein erstreckt.

13. Befestigungssystem für Referenzanordnungen gemäß einem der vorhergehenden Ansprüche, **dadurch gekennzeichnet, dass** die Referenzanordnung (2) von der Ansatzbasis (3) abnehmbar ist.

14. Navigationssystem (13) mit zumindest einer Referenzanordnung (2), die gemäß einem der vorhergehenden Ansprüche an einem Knochen (4) fixiert wird.

15. Verfahren zum Befestigen einer Referenzanordnung an einem Knochen (4) mit einem verflüssigbaren Material (1), das eine der Referenzanordnung (2) zugeordnete Ansatzbasis (3) gegenüber einem Knochen (4) fixiert, mit zumindest einem der folgenden Schritte:
- Ansetzen der Ansatzbasis (3) in oder an einen Knochen (4), insbesondere in oder an eine erzeugte Aufnahme, im speziellen auf die bearbeitete Knochenoberfläche (6) oder in ein im Knochen vorgebohrtes Sackloch (8).
- Verflüssigen des Materials (1), insbesondere durch zumindest einen der folgenden auf das Material (1) einwirkenden Einfluss:
- Ultraschallwellen
- Thermische Energie
- Infrarotstrahlung
- Mikrowellenstrahlung
- UV-Strahlung,
- Verbringen des viskosen Materials (1), insbesondere durch einen in der Ansatzbasis (3) angeordneten Kanal (9) in einen an Ansatzbasis (3) und Knochen (4) anliegenden Bereich (5),
- Aushärten des Materials (1)
- Entfernen der Ansatzbasis (3) vom Knochen (4).

16. Verfahren gemäß Anspruch 14, wobei zum Entfernen der Ansatzbasis (3) das Material (1) wieder verflüssigt wird, insbesondere durch zumindest eine der folgenden auf das Material (1) einwirkenden Größen:
- Ultraschallwellen
- Thermische Energie
- Infrarotstrahlung
- Mikrowellenstrahlung
- UV-Strahlung

17. Verfahren gemäß Anspruch 14, wobei zum Entfernen der Ansatzbasis (3) die Ansatzbasis (3) mechanisch abgetrennt wird, insbesondere so, dass der am Knochen (4) verbleibende Teil der Ansatzbasis (3) bündig mit dem Knochen (4) abschließt.

18. Verfahren gemäß Anspruch 14, wobei zum Entfernen der Ansatzbasis (3) zumindest ein Teil des Materials (1) mechanisch entfernt wird, insbesondere durch Ausbohren eines runden Zuführkanals (9) in der Ansatzbasis (3).
